# EUROPEAN PATENT APPLICATION

(11) **EP 4 353 723 A1**
(43) Date of publication of application: **17.04.2024**
(21) Application number: 22819443.7
(22) Date of filing: 02.06.2022
(51) Int. Cl.: C07D 471/04, A61K 31/437, A61K 31/4545, A61P 35/00, A61P 35/02, A61P 37/00, A61P 25/00, A61P 37/06

(54) **CRYSTAL FORM OF TOLEBRUTINIB, PREPARATION METHOD THEREFOR AND USE THEREOF**

(30) Priority: 11.06.2021 CN 202110652005; 22.06.2021 CN 202110690510; 10.11.2021 CN 202111326112
(71) Applicant: GENZYME CORPORATION, Cambridge, Massachusetts 02141 (US)
(72) Inventor: CHEN, Minhua, Suzhou, Jiangsu 215123 (CN); SHI, Jiaming, Suzhou, Jiangsu 215123 (CN); ZHANG, Jing, Suzhou, Jiangsu 215123 (CN)
(74) Representative: Cabinet Nony
(86) International application number: PCT/CN2022/096779
(87) International publication number: WO 2022/257845

(57) **Abstract**

The present invention relates to a new crystal form of tolebrutinib (hereinafter referred to as "compound I"), a preparation method therefor, a pharmaceutical composition containing the crystal form, and the use of same in the preparation of a BTK inhibitor drug and a drug for treating multiple sclerosis. The crystal form of tolebrutinib provided has one or more improved properties compared with the prior art, and is of great value to the optimization and development of drugs in the future.

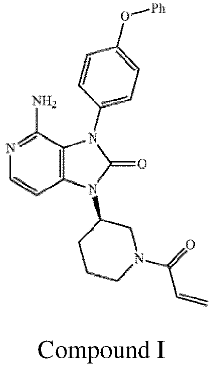

## Description

### TECHNICAL FIELD

The present disclosure pertains to the field of chemical crystallography, particularly relates to crystalline forms of Tolebrutinib, preparation method and use thereof.

### BACKGROUND

Multiple Sclerosis (MS) is a neurological disease affecting more than 1 million people worldwide. It is the most common cause of neurological disability in young and middle-aged adults and has a major physical, psychological, social and financial impact on subjects and their families. MS involves an immune-mediated process in which an abnormal response of the body's immune system is directed against the central nervous system (CNS). In the course of the disease, scleroses, i.e., lesions or scars, appear in the myelin sheath of nerve cells, disrupting transmission of electrical signals. Scleroses accumulate over time and result in the debilitating symptoms experienced by MS patients.

Immunomodulatory drugs have been the mainstay of MS therapy. Results from a clinical study in 2017 (Hauser et al., N Engl JMed. 2017; 376 (3):221-34) has demonstrated efficacy of agents that target B lymphocytes.

The Bruton's tyrosine kinase (BTK) pathway is critical to signaling in B lymphocytes and myeloid cells including CNS microglia. Each of these cell types has been implicated in the pathophysiology of MS. Further, as BTK signaling is vital for maturation of B cells into antibody-secreting plasma cells, BTK inhibition can modulate both cellular and humoral immunity. Accordingly, an inhibitor of BTK signaling represents a dual mechanism targeting both aspects of the immune system.

Accordingly, compounds that inhibit BTK that are able to both inhibit antigen-induced B-cell activation responsible for neuroinflammation and modulate maladaptive microglia cells linked to neuroinflammation in the brain and spinal cord may be useful in treating relapsing multiple sclerosis (RMS) with superior benefits when compared to currently available therapies.

Tolebrutinib, an oral selective BTK inhibitor, has shown safety and efficacy in patients with RMS.

The chemical name of Tolebrutinib is (R)-1-(1-acryloylpiperidin-3-yl)-4-amino-3-(4-phenoxyphenyl)-1H-imidazo[4,5-c] pyridin-2(3H)-one (hereinafter referred to as Compound I), and the structure is shown as follows:

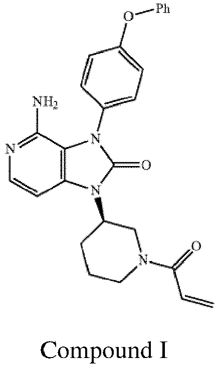

A crystalline form is a solid material whose constituents are arranged in a highly ordered microscopic structure, forming a crystal lattice that extends in all directions. Polymorphism refers to the phenomenon that a compound exists in more than one crystalline form. Compounds may exist in one or more crystalline forms, but their existence and characteristics cannot be predicted with any certainty. Different crystalline forms of drug substances have different physicochemical properties, which can affect drug's in vivo dissolution and absorption and will further affect drug's clinical efficacy to some extent. In particular, for some poorly soluble oral solid or semi-solid dosage forms, crystalline forms can be crucial to the performance of drug product. In addition, the physiochemical properties of a crystalline form are very important to the production process. Therefore, polymorphism is an important part of drug research and drug quality control.

A white solid of Compound I was disclosed in WO2016196840A1. The inventors of the present disclosure repeated the preparation process and an amorphous of Compound I was obtained. Furthermore, the inventors of the present disclosure have studied the amorphous obtained, and the results show that the amorphous of Compound I has disadvantages such as poor stability, strong hygroscopicity and easy degradability, and is not suitable for medicine use.

In order to overcome the disadvantages of prior arts, there is still a need for a new crystalline form that meets pharmaceutical standards for the development of drugs containing Compound I. The inventors of present disclosure surprisingly obtained a crystalline form of Compound I, which has advantages in aspects of solubility, hygroscopicity, purification ability, stability, adhesiveness, compressibility, flowability, in vitro and in vivo dissolution, bioavailability, etc. In particular, the crystalline form of Compound I of the present disclosure has advantages such as good stability, lower hygroscopicity, and little degradability, which solves the problems existing in the prior art and is of great significance for the development of drugs containing Compound I.

### SUMMARY

The present disclosure is to provide a novel crystalline form of Compound I, preparation method and use thereof, and pharmaceutical compositions containing the novel crystalline form.

According to the objective of the present disclosure, crystalline form CSII of Compound I is provided (hereinafter referred to as Form CSII).

In one aspect provided herein, the X-ray powder diffraction pattern of Form CSII comprises characteristic peaks at 2theta values of 4.1°±0.2°, 10.2°±0.2° and 22.6°±0.2° using CuKα radiation.

Furthermore, the X-ray powder diffraction pattern of Form CSII comprises one or two or three characteristic peaks at 2theta values of 11.3°±0.2°, 16.5°±0.2° and 17.8°±0.2° using CuKα radiation. Preferably, the X-ray powder diffraction pattern of Form CSII comprises characteristic peaks at 2theta values of 11.3°±0.2°, 16.5°±0.2° and 17.8°±0.2° using CuKα radiation.

Furthermore, the X-ray powder diffraction pattern of Form CSII comprises one or two or three characteristic peaks at 2theta values of 8.2°±0.2°, 10.8°±0.2° and 24.7°±0.2° using CuKα radiation. Preferably, the X-ray powder diffraction pattern of Form CSII comprises characteristic peaks at 2theta values of 8.2°±0.2°, 10.8°±0.2° and 24.7°±0.2° using CuKα radiation.

In another aspect provided herein, the X-ray powder diffraction pattern of Form CSII comprises one or two or three or four or five or six or seven or eight or nine or ten characteristic peaks at 2theta values of 4.1°±0.2°, 10.2°±0.2°, 22.6°±0.2°, 11.3°±0.2°, 16.5°±0.2°, 17.8°±0.2°, 8.2°±0.2°, 10.8°±0.2°, 24.7°±0.2° and 20.5°±0.2° using CuKα radiation.

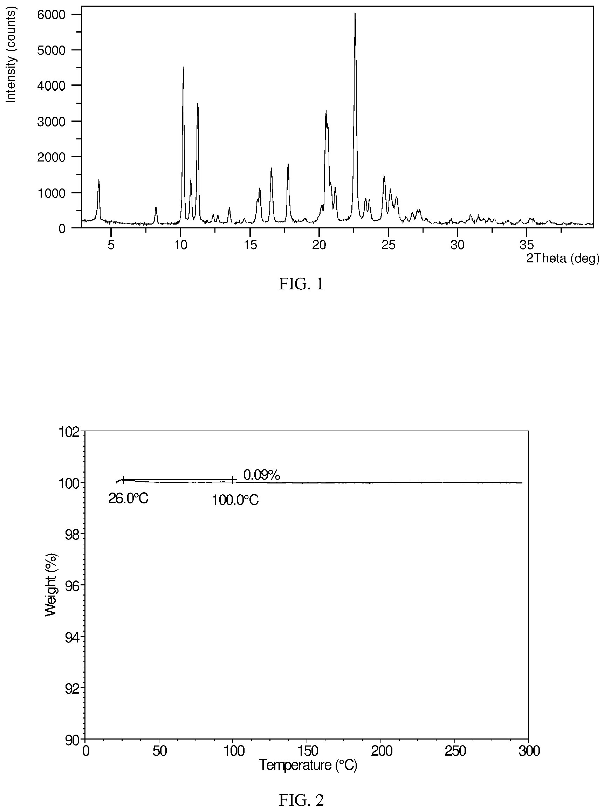

Without any limitation being implied, the X-ray powder diffraction pattern of Form CSII is substantially as depicted in Figure 1 using CuKα radiation.

Without any limitation being implied, the TGA curve of Form CSII is substantially as depicted in Figure 2, which shows about 0.1% weight loss when heated from 26 °C to 100 °C.

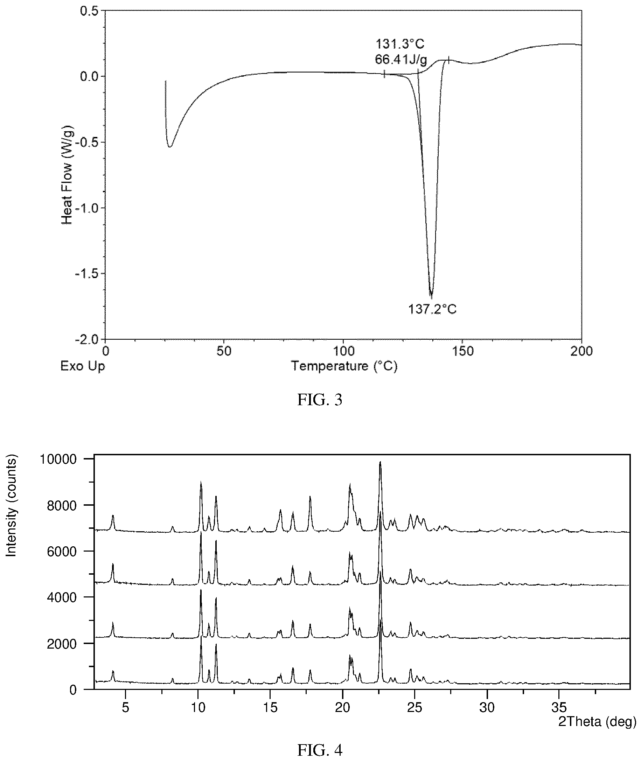

Without any limitation being implied, the DSC curve of Form CSII is substantially as depicted in Figure 3, which shows an endothermic peak at around 131 °C (onset temperature). This peak is the melting endothermic peak.

Without any limitation being implied, Form CSII is an anhydrate.

According to the objective of the present disclosure, a process for preparing Form CSII is also provided. The process comprises:
Adding the solid of Compound I into an alcohol solvent to form a suspension, stirring at a certain temperature and separating to obtain solid, drying the solid by high temperature vaccum drying for a certain time to obtain Form CSII;
Furthermore, said alcohol solvent is preferably an alcohol of C 1-C4, more preferably ethanol; said temperature is preferably 0-50°C, more preferably 50°C; said stirring time is preferably more than 1 day; temperature of said high temperature vaccum drying is preferably 50-75°C; said drying time is more than 3 hours.

According to the objective of the present disclosure, the present disclosure provides Form CSIII of Compound I (hereinafter referred to as Form CSIII).

In one aspect provided herein, the X-ray powder diffraction pattern of Form CSIII comprises characteristic peaks at 2theta values of 4.2°±0.2°, 11.1°±0.2° and 21.7°±0.2° using CuKα radiation.

Furthermore, the X-ray powder diffraction pattern of Form CSIII comprises one or two or three characteristic peaks at 2theta values of 20.6°±0.2°, 21.0°±0.2° and 22.2°±0.2° using CuKα radiation. Preferably, the X-ray powder diffraction pattern of Form CSIII comprises characteristic peaks at 2theta values of 20.6°±0.2°, 21.0°±0.2° and 22.2°±0.2° using CuKα radiation.

Furthermore, the X-ray powder diffraction pattern of Form CSIII comprises one or two or three characteristic peaks at 2theta values of 10.4°±0.2°, 17.7°±0.2° and 23.1°±0.2° using CuKα radiation. Preferably, the X-ray powder diffraction pattern of Form CSIII comprises characteristic peaks at 2theta values of 10.4°±0.2°, 17.7°±0.2° and 23.1°±0.2° using CuKα radiation.

In another aspect provided herein, the X-ray powder diffraction pattern of Form CSIII comprises one or two or three or four or five or six or seven or eight or nine or ten or eleven or twelve or thirteen or fourteen characteristic peaks at 2theta values of 4.2°±0.2°, 11.1°±0.2°, 21.7°±0.2°, 20.6°±0.2°, 21.0°±0.2°, 22.2°±0.2°, 10.4°±0.2°, 17.7°±0.2°, 23.1°±0.2°, 8.4°±0.2°, 13.3°±0.2°, 16.3°±0.2°, 24.2°±0.2°, and 25.4°±0.2° using CuKα radiation.

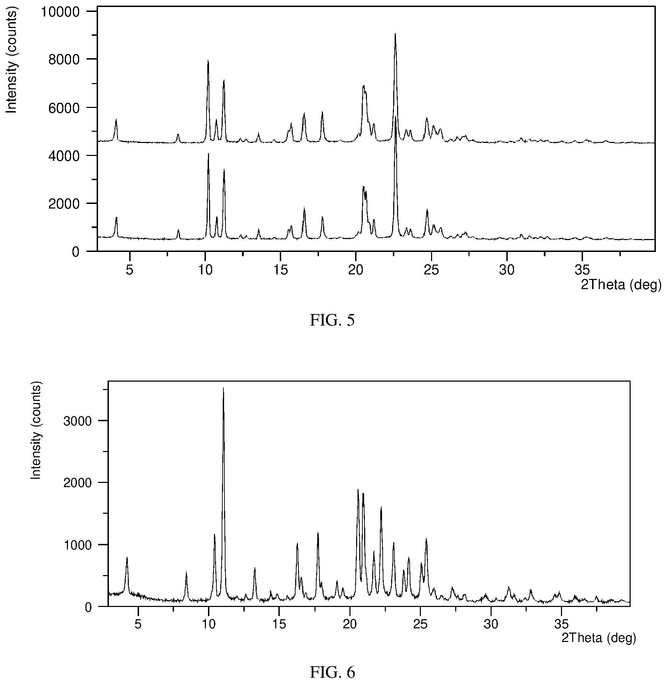

Without any limitation being implied, the X-ray powder diffraction pattern of Form CSIII is substantially as depicted in Figure 6 using CuKα radiation.

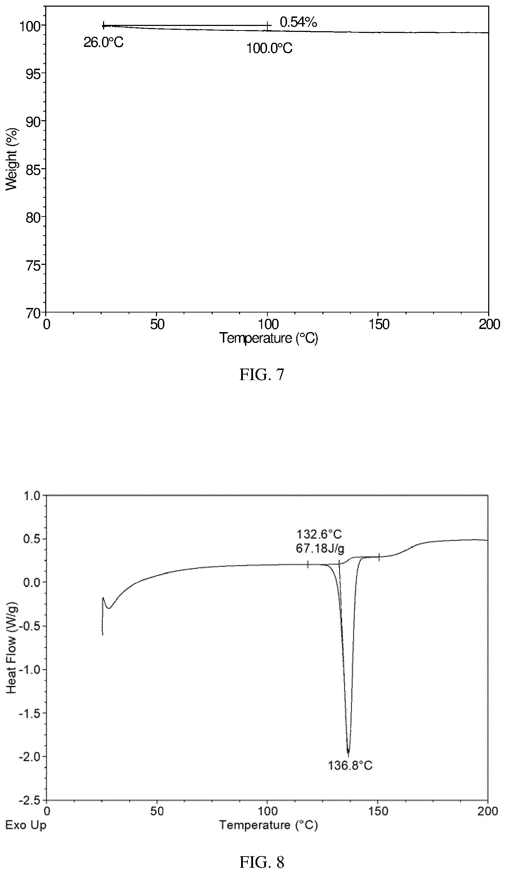

Without any limitation being implied, the TGA curve of Form CSIII is substantially as depicted in Figure 7, which shows about 0.6% weight loss when heated from 26 °C to 100 °C.

Without any limitation being implied, the DSC curve of Form CSIII is substantially as depicted in Figure 8, which shows an endothermic peak at around 133 °C (onset temperature). This peak is the melting endothermic peak.

Without any limitation being implied, Form CSIII is an anhydrate.

According to the objective of the present disclosure, a process for preparing Form CSIII is also provided. The process comprises:
Adding the solid of Compound I into acetone to form a suspension, stirring to obtain Form CSIII;
Furthermore, temperature of said stirring is preferably 0-50°C, more preferably 5°C.

According to the objective of the present disclosure, the present disclosure provides Form CSIV of Compound I (hereinafter referred to as Form CSIV).

In one aspect provided herein, the X-ray powder diffraction pattern of Form CSIV comprises characteristic peaks at 2theta values of 8.5°±0.2°, 18.6°±0.2° and 22.0°±0.2° using CuKα radiation.

Furthermore, the X-ray powder diffraction pattern of Form CSIV comprises one or two or three characteristic peaks at 2theta values of 12.9°±0.2°, 19.1°±0.2° and 23.3°±0.2° using CuKα radiation. Preferably, the X-ray powder diffraction pattern of Form CSIV comprises characteristic peaks at 2theta values of 12.9°±0.2°, 19.1°±0.2° and 23.3°±0.2° using CuKα radiation.

Furthermore, the X-ray powder diffraction pattern of Form CSIV comprises one or two or three characteristic peaks at 2theta values of 13.2°±0.2°, 13.8°±0.2° and 21.1°±0.2° using CuKα radiation. Preferably, the X-ray powder diffraction pattern of Form CSIV comprises characteristic peaks at 2theta values of 13.2°±0.2°, 13.8°±0.2° and 21.1°±0.2° using CuKα radiation.

In another aspect provided herein, the X-ray powder diffraction pattern of Form CSIV comprises one or two or three or four or five or six or seven or eight or nine or ten or eleven or twelve characteristic peaks at 2theta values of 8.5°±0.2°, 18.6°±0.2°, 22.0°±0.2°, 12.9°±0.2°, 19.1°±0.2°, 23.3°±0.2°, 13.2°±0.2°, 13.8°±0.2°, 21.1°±0.2°, 7.7°±0.2°, 17.2°±0.2° and 26.7°±0.2° using CuKα radiation.

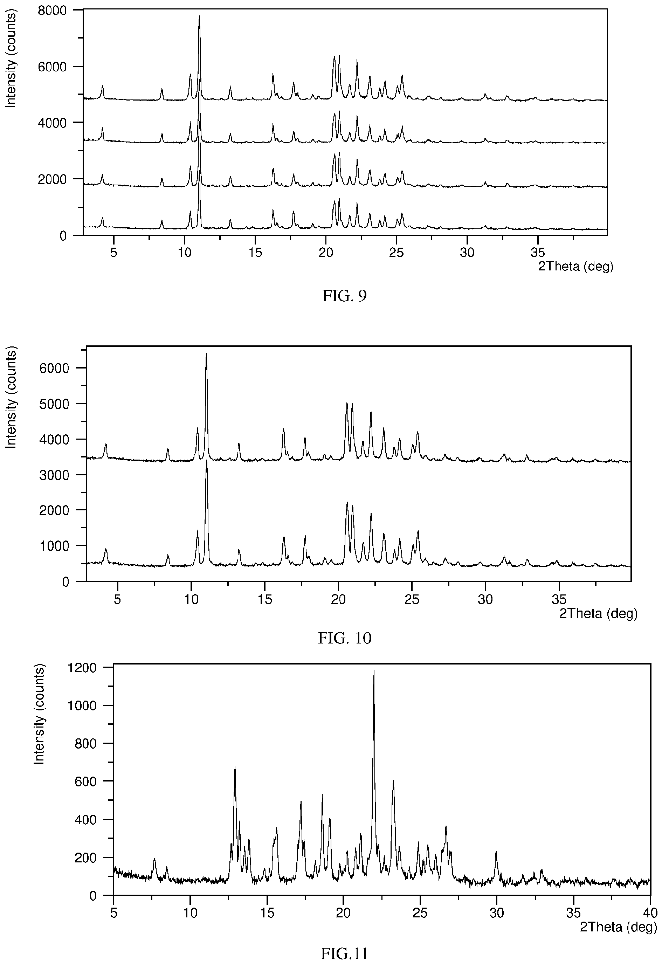

Without any limitation being implied, the X-ray powder diffraction pattern of Form CSIV is substantially as depicted in Figure 11 using CuKα radiation.

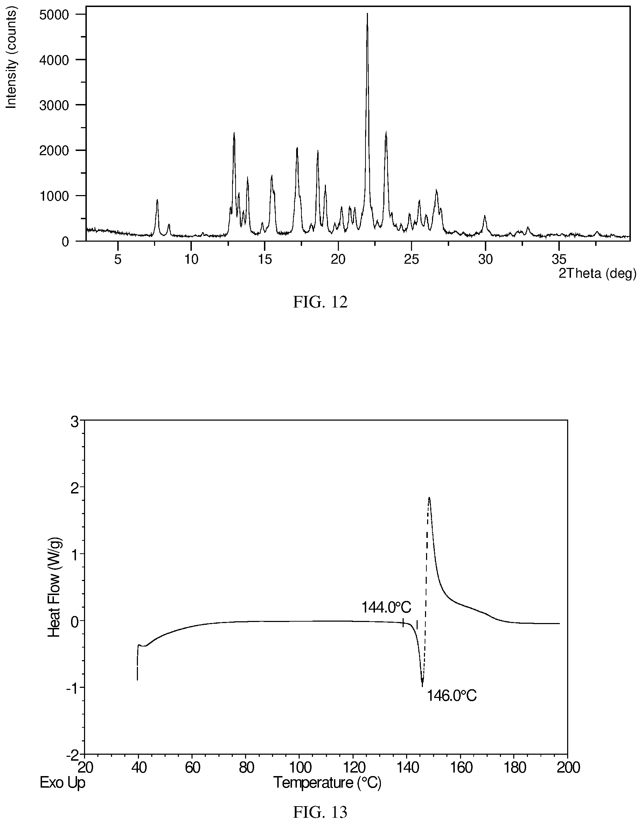

Without any limitation being implied, the DSC curve of Form CSIV is substantially as depicted in Figure 13, which shows an endothermic peak at around 144 °C (onset temperature). This peak is the melting endothermic peak.

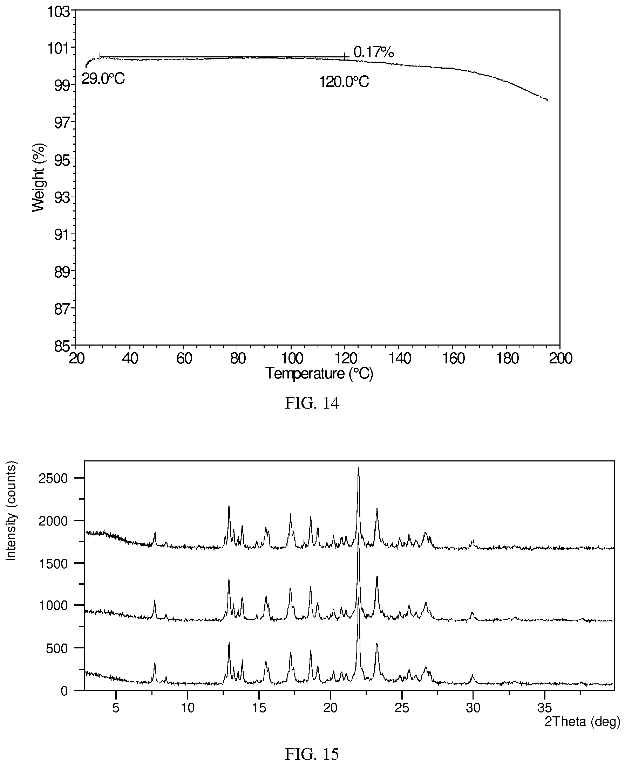

Without any limitation being implied, the TGA curve of Form CSIV is substantially as depicted in Figure 14, which shows about 0.2% weight loss when heated from 29 °C to 120 °C.

Without any limitation being implied, Form CSIV is an anhydrate.

According to the objective of the present disclosure, a process for preparing Form CSIV is also provided. The process comprises:
Adding the solid of Compound I into an ether solvent or an aromatic hydrocarbon solvent to form a suspension, stirring at -20°C -5°C to obtain Form CSIV of the present invention;
Furthermore, said ether solvent is preferably an ether of C5, more preferably methyl tert-butyl ether; said aromatic hydrocarbon solvent is preferably an aromatic hydrocarbon solvent of C9, more preferably isopropyl benzene; said stirring temperature is preferably -20°C.

According to the objective of the present disclosure, the present disclosure provides the use of Form CSII, Form CSIII, Form CSIV, or any mixture of any two crystalline forms, or any mixture of three crystalline forms, for preparing other crystalline forms, or salts of Compound I.

According to the objective of the present disclosure, a pharmaceutical composition is provided, said pharmaceutical composition comprises a therapeutically effective amount of Form CSII, Form CSIII, Form CSIV, or any mixture of the three crystalline forms and pharmaceutically acceptable excipients.

Furthermore, use of Form CSII, Form CSIII, Form CSIV, or any mixture of any two crystalline forms, or any mixture of three crystalline forms is provided by present disclosure for the preparation of a BTK inhibitor drug.

Furthermore, use of Form CSII, Form CSIII, Form CSIV, or any mixture of any two crystalline forms, or any mixture of three crystalline forms is provided by present disclosure for the preparation of a drug for the treatment of multiple sclerosis.

### Technical effects

Form CSII drug substance of the present disclosure has the following unexpected advantages:
(1) Form CSII drug substance of the present disclosure has better stability than the prior art.

The chemical purity of the prior art solid decreases significantly when stored under the conditions of 25 °C/60%RH, 40 °C/75%RH, 60 °C/75%RH, and 80 °C with light shedding. In particular, after storage at 40 °C/75%RH for 6 month, the purity decreases by 3.46%, and the number of impurities which exceed the qualificated threshold increases to four. After storage at 60 °C/75%RH for only 1 month, the purity decreases over 6.3%, and the number of impurities which exceed the qualificated threshold increases to four. The chemical stability of the prior art solid is far below the medicinal standard.

Crystalline state of Form CSII drug substance of the present disclosure doesn't change for at least 6 months when stored under the condition of 25 °C/60%RH. The chemical purity is above 99.8% and the crystalline form remains substantially unchanged during storage. These results show that From CSII drug substance of the present disclosure has good stability under long term condition which is suitable for drug storage.

Meanwhile, crystalline state of Form CSII drug substance doesn't change for at least 6 months when stored under the condition of 40 °C/75%RH. The crystalline state of Form CSII drug substance doesn't change for at least 1 month when stored under the condition of 60 °C/75%RH. The chemical purity is above 99.8% and remains substantially unchanged during storing. The chemical purity of Form CSII drug substance remains substantially unchanged for at least 2 days when stored under the condition of 80 °C.

Under the condition that the total illuminance of the light source is not less than 1.2×10⁶lux-hr, and the energy of the near-ultraviolet lamp is not less than 200W-hr/m² energy, there is no change in the purity for at least 1 week. These results show that Form CSII drug substance has better stability under accelerated, high temperature and lighting conditions.

High temperature and high humidity conditions caused by different season, regional climate and environment, etc. will affect storage, transportation, and manufacturing processes of drug substance and drug product. During storage, transportation, and manufacturing processes of the drug substances, the influence of light condition inevitably exists, therefore, good stability under accelerated, high temperature and lighting conditions is of great importance to the drug development. Form CSII drug substance has good stability under accelerated, high temperature and lighting conditions, which is beneficial to avoid the impact on drug quality due to crystal transformation or decrease in purity during drug storage.

In addition, the impurity content of Form CSII drug substance did not exceed the qualificated threshold throughout the stability investigation processes, which can meet the requirements of pharmaceutical development.

Good physical and chemical stability of drug substances ensure that during production and storage, no crystal transformation occurs, and substantially no impurity is generated. Form CSII has good physical and chemical stability, ensuring consistent and controllable quality of the drug substance and drug product, minimizing quality changes, bioavailability changes, toxicity and side effects caused by crystal transformation or impurity generation.

In addition, the crystalline form of Form CSII did not change after it was mixed with the excipients to form a drug product, indicating that the Form CSII drug product is stable during the preparation process, which is favorable for the production of drugs.

Furthermore, Form CSII has good physical stability under mechanical force. Form CSII remains unchanged after grinding of the drug substance. It is often necessary to grind or pulverize the drug substance during preparation process, and good physical stability can reduce the risk of decreased crystallinity and crystal transformation of the drug substance during preparation process.

(2) Compared with prior art, Form CSII of the present disclosure has lower hygroscopicity. The test results show that the weight gain of Form CSII is only 1/6 that of the prior art. The weight gain of Form CSII at 80%RH is 0.60%, indicating that Form CSII is slightly hygroscopic. The weight gain of the prior art solid at 80%RH is 3.69%, indicating that the prior art is hygroscopic. In one aspect, high hygroscopicity tends to cause chemical degradation and polymorph transformation, which directly affects the physical and chemical stability of the drug substance. In addition, high hygroscopicity will reduce the flowability of the drug substance, thereby affecting the processing of the drug substance.

In another aspect, drug substance with high hygroscopicity requires low humidity environment during production and storage, which puts strict requirements on production and imposes higher costs. More importantly, high hygroscopicity is likely to cause variation in the content of active pharmaceutical ingredients in the drug product, thus affecting drug product quality.

Form CSII provided by the present disclosure with lower hygroscopicity is not demanding on the production and storage conditions, which reduces the cost of production, storage and quality control, and has strong economic value.

Form CSIII drug substance of the present disclosure has the following unexpected advantages:
(1) Form CSIII drug substance of the present disclosure has better stability than the prior art. The chemical purity of the prior art solid decreases significantly when stored under the conditions of 25 °C/60%RH, 40 °C/75%RH, 60 °C/75%RH, and 80 °C with light shedding. In particular, after storage at 40 °C/75%RH for 6 month, the purity decreases by 3.46%, and the number of impurities which exceed the qualificated threshold increases to four. After storage at 60 °C/75%RH for only 1 month, the purity decreases over 6.3%, and the number of impurities which exceed the qualificated threshold increases to four. The chemical stability of the prior art solid is far below the medicinal standard.

Crystalline state of Form CSIII drug substance of the present disclosure doesn't change for at least 6 months when stored under the condition of 25 °C/60%RH. The chemical purity is above 99.9% and the crystalline from remains substantially unchanged during storage. These results show that From CSIII drug substance of the present disclosure has good stability under long term condition which is suitable for drug storage.

Meanwhile, crystalline state of Form CSIII drug substance doesn't change for at least 6 months when stored under the condition of 40 °C/75%RH. The crystalline state of Form CSIII drug substance doesn't change for at least 1 month when stored under the condition of 60 °C/75%RH. The chemical purity is above 99.8% and remains substantially unchanged during storing. The chemical purity of Form CSIII drug substance remains substantially unchanged for at least 2 days when stored under the condition of 80 °C.

Under the condition that the total illuminance of the light source is not less than 1.2×10⁶lux-hr, and the energy of the near-ultraviolet lamp is not less than 200W-hr/m² energy, there is no change in the purity for at least 1 week. These results show that Form CSIII drug substance has better stability under accelerated, high temperature and lighting conditions. High temperature and high humidity conditions caused by different season, regional climate and environment, etc. will affect storage, transportation, and manufacturing processes of drug substance and drug product. During storage, transportation, and manufacturing processes of the drug substances, the influence of light condition inevitably exists, therefore, good stability under accelerated, high temperature and lighting conditions is of great importance to the drug development. Form CSIII drug substance has good stability under accelerated, high temperature and lighting conditions, which is beneficial to avoid the impact on drug quality due to crystal transformation or decrease in purity during drug storage.

In addition, the impurity content of Form CSIII drug substance did not exceed the qualificated threshold throughout the stability investigation processes, which can meet the requirements of pharmaceutical development.

Good physical and chemical stability of drug substances ensure that during production and storage, no crystal transformation occurs, and substantially no impurity is generated. Form CSIII has good physical and chemical stability, ensuring consistent and controllable quality of the drug substance and drug product, minimizing quality changes, bioavailability changes, toxicity and side effects caused by crystal transformation or impurity generation.

In addition, the crystalline form of Form CSIII did not change after it was mixed with the excipients to form a drug product, indicating that the Form CSIII drug product is stable during the preparation process, which is favorable for the production of drugs.

Furthermore, Form CSIII has good physical stability under mechanical force. Form CSIII remains unchanged after grinding of the drug substance. It is often necessary to grind or pulverize the drug substance during preparation process, and good physical stability can reduce the risk of decreased crystallinity and crystal transformation of the drug substance during preparation process.

(2) Compared with prior art, Form CSIII of the present disclosure has lower hygroscopicity. The test results show that the weight gain of Form CSIII is only 1/6 that of the prior art. The weight gain of Form CSIII at 80%RH is 0.66%, indicating that Form CSIII is slightly hygroscopic. The weight gain of the prior art solid at 80%RH is 3.69%, indicating that the prior art is hygroscopic. In one aspect, high hygroscopicity tends to cause chemical degradation and polymorph transformation, which directly affects the physical and chemical stability of the drug substance. In addition, high hygroscopicity will reduce the flowability of the drug substance, thereby affecting the processing of the drug substance.

In another aspect, drug substance with high hygroscopicity requires low humidity environment during production and storage, which puts strict requirements on production and imposes higher costs. More importantly, high hygroscopicity is likely to cause variation in the content of active pharmaceutical ingredients in the drug product, thus affecting drug product quality.

Form CSIII provided by the present disclosure with lower hygroscopicity is not demanding on the production and storage conditions, which reduces the cost of production, storage and quality control, and has strong economic value.

Form CSIV drug substance of the present disclosure has the following unexpected advantages:
(1) Form CSIV drug substance of the present disclosure has better stability than the prior art. The chemical purity of the prior art solid decreases significantly when stored under the condition of 40 °C/75%RH for 2 months, the purity decreases by 2.18%.

Crystalline state of Form CSIV drug substance of the present disclosure doesn't change for at least 2 months when stored under the condition of 25 °C/60%RH. The chemical purity is above 99.7% and the crystalline form remains substantially unchanged during storage. These results show that From CSIV drug substance of the present disclosure has good stability under long term condition which is suitable for drug storage.

Meanwhile, crystalline state of Form CSIV drug substance doesn't change for at least 2 months when stored under the condition of 40 °C/75%RH. These results show that Form CSIV drug substance has better stability under accelerated conditions. High temperature and high humidity conditions caused by different season, regional climate and environment, etc. will affect storage, transportation, and manufacturing processes of drug substance and drug product. Therefore, good stability under accelerated conditions is of great importance to the drug development. Form CSIV drug substance has good stability under accelerated conditions, which is beneficial to avoid the impact on drug quality due to crystal transformation or decrease in purity during drug storage. Good physical and chemical stability of drug substances ensure that during production and storage, no crystal transformation occurs, and substantially no impurity is generated. Form CSIV has good physical and chemical stability, ensuring consistent and controllable quality of the drug substance and drug product, minimizing quality changes, bioavailability changes, toxicity and side effects caused by crystal transformation or impurity generation.

In addition, the crystalline form of Form CSIV did not change after it was mixed with the excipients to form a drug product, indicating that the Form CSIV drug product is stable during the preparation process, which is favorable for the production of drugs.

Furthermore, Form CSIV has good physical stability under mechanical force. Form CSIV remains unchanged after grinding of the drug substance. It is often necessary to grind or pulverize the drug substance during preparation process, and good physical stability can reduce the risk of decreased crystallinity and crystal transformation of the drug substance during preparation process.

(2) Compared with prior art, Form CSIV of the present disclosure has lower hygroscopicity. The test results show that the weight gain of Form CSIV is only 1/15 that of the prior art. The weight gain of Form CSIV at 80%RH is 0.24%, indicating that Form CSIV is slightly hygroscopic. The weight gain of the prior art solid at 80%RH is 3.69%, indicating that the prior art is hygroscopic. In one aspect, high hygroscopicity tends to cause chemical degradation and polymorph transformation, which directly affects the physical and chemical stability of the drug substance. In addition, high hygroscopicity will reduce the flowability of the drug substance, thereby affecting the processing of the drug substance.

In another aspect, drug substance with high hygroscopicity requires low humidity environment during production and storage, which puts strict requirements on production and imposes higher costs. More importantly, high hygroscopicity is likely to cause variation in the content of active pharmaceutical ingredients in the drug product, thus affecting drug product quality.

Form CSIV provided by the present disclosure with lower hygroscopicity is not demanding on the production and storage conditions, which reduces the cost of production, storage and quality control, and has strong economic value.

### BRIEF DESCRIPTION OF THE DRAWINGS

Figure 1 shows an XRPD pattern of Form CSII.
Figure 2 shows a TGA curve of Form CSII.
Figure 3 shows a DSC curve of Form CSII.
Figure 4 shows an XRPD pattern overlay of Form CSII before and after storage (from top to bottom: initial, stored at 25 °C/60%RH (open package) for 6 months, stored at 40 °C/75%RH (open package) for 6 months, stored at 60 °C/75%RH (open package) for 1 month).
Figure 5 shows an XRPD pattern overlay of Form CSII before and after DVS test (from top to bottom: initial, final).
Figure 6 shows an XRPD pattern of Form CSIII.
Figure 7 shows a TGA curve of Form CSIII.
Figure 8 shows a DSC curve of Form CSIII.
Figure 9 shows an XRPD pattern overlay of Form CSIII before and after storage (from top to bottom: initial, stored at 25 °C/60%RH (open package) for 6 months, stored at 40 °C/75%RH (open package) for 6 months, stored at 60 °C/75%RH (open package) for 1 month).
Figure 10 shows an XRPD pattern overlay of Form CSIII before and after DVS test (from top to bottom: initial, final).
Figure 11 shows an XRPD pattern of Form CSIV.
Figure 12 shows an XRPD pattern of Form CSIV.
Figure 13 shows a TGA curve of Form CSIV.
Figure 14 shows a DSC curve of Form CSIV.
Figure 15 shows an XRPD pattern overlay of Form CSIV before and after storage (from top to bottom: initial, stored at 25 °C/60%RH (open package) for 6 months, stored at 40 °C/75%RH (open package) for 6 months).
Figure 16 shows an XRPD pattern overlay of Form CSIV before and after DVS test (from top to bottom: initial, final).

### DETAILED DESCRIPTION

The present disclosure is further illustrated by the following examples which describe the preparation and use of the crystalline forms of the present disclosure in detail. It is obvious to those skilled in the art that changes in the materials and methods can be accomplished without departing from the scope of the present disclosure.

The abbreviations used in the present disclosure are explained as follows:
XRPD: X-ray Powder Diffraction
DSC: Differential Scanning Calorimetry
TGA: Thermo Gravimetric Analysis
DVS: Dynamic Vapor Sorption
¹H NMR: Proton Nuclear Magnetic Resonance
RH: Relative humidity
UPLC: Ultra Performance Liquid Chromatography
LC: Liquid Chromatography
TEA: Triethylamine

Instruments and methods used for data collection:
X-ray powder diffraction patterns in the present disclosure were acquired by a Bruker X-ray powder diffractometer. The parameters of the X-ray powder diffraction method of the present disclosure are as follows:
X-Ray: Cu, Kα
Kα1 (Å): 1.54060; Kα2 (Å): 1.54439
Kα2/Kα1 intensity ratio: 0.50

TGA data in the present disclosure were acquired by a TA Q500. The parameters of the TGA method of the present disclosure are as follows:
Heating rate: 10 °C/ min
Purge gas: nitrogen

DSC data in the present disclosure were acquired by a TA Q2000. The parameters of the DSC method of the present disclosure are as follows:
Heating rate: 10 °C/min
Purge gas: nitrogen

DVS was measured via an SMS (Surface Measurement Systems Ltd.) intrinsic DVS instrument. Parameters for DVS method are as follows:
Temperature: 25 °C
Gas and flow rate: nitrogen, 200 mL/min
RH range: 0%RH to 95%RH
¹H NMR were collected from a Bruker Avance II DMX 400M HZ NMR spectrometer. 1-5 mg of sample was weighed and dissolved in 0.5 mL of deuterated dimethyl sulfoxide to obtain a solution with a concentration of 2-10 mg/mL.

The related substance in the present disclosure was detected by UPLC and the parameters are shown below.

**Table 1**

| | | |
|---|---|---|
| Instrument | Waters ACQUITY UPLC H-Class with PDA | |
| Column | ACE Excel 3 C18 | |
| Mobile phase | A:0.1% H₃PO₄ in H₂O (pH4.0, TEA) | |
| | B: Acetonitrile | |

| Gradient | Time (min) | %B |
|---|---|---|
| | 0.0 | 10 |
| | 0.3 | 10 |
| | 3.5 | 45 |
| | 9.0 | 80 |
| | 11.0 | 80 |
| | 11.1 | 10 |
| | 18.0 | 10 |
| Run time | 18.0 min | |
| Stop time | 0.0 min | |
| Injection volume | 1 µL | |
| Detector wavelength | 226 nm | |
| Column temperature | 40 °C | |
| Sample temperature | Room temperature | |
| Diluent | 0.01% TFA in Acetonitrile | |

In the present disclosure, said "stirring" is accomplished by using a conventional method in the field such as magnetic stirring or mechanical stirring and the stirring speed is 50 to 1800 r/min. Preferably the magnetic stirring speed is 300 to 900 r/min and mechanical stirring speed is 100 to 300 r/min.

Said "separation" is accomplished by using a conventional method in the field such as centrifugation or filtration. The operation of "centrifugation" is as follows: the sample to be separated is placed into the centrifuge tube, and then centrifuged at a rate of 10000 r/min until the solid all sink to the bottom of the tube.

Said "drying" is accomplished by using a conventional method in the field such as vacuum drying, blast drying or free-air drying. The drying temperature can be room temperature or higher. Preferably the drying temperature is from room temperature to about 60 °C, or to 50 °C, or to 40 °C. The drying time can be 2 to 48 hours, or overnight. Drying is accomplished in a fume hood, forced air convection oven or vacuum oven.

Said "room temperature" is not a specific temperature, but a temperature range of 10-30 °C. Said "open packaged" is putting the sample into a glass vial, covering the vial with aluminum foil, and punching 5-10 holes on the foil.

Said "characteristic peak" refers to a representative diffraction peak used to distinguish crystals, which usually can have a deviation of ±0.2° using CuKα radiation.

In the present disclosure, "crystal" or "crystalline form" refers to the crystal or the crystalline form being identified by the X-ray diffraction pattern shown herein. Those skilled in the art are able to understand that the experimental errors depend on the instrument conditions, the sample preparation and the purity of samples. The relative intensity of the diffraction peaks in the X-ray diffraction pattern may also vary with the experimental conditions; therefore, the order of the diffraction peak intensities cannot be regarded as the sole or decisive factor. In fact, the relative intensity of the diffraction peaks in the X-ray powder diffraction pattern is related to the preferred orientation of the crystals, and the diffraction peak intensities shown herein are illustrative and identical diffraction peak intensities are not required. Thus, it will be understood by those skilled in the art that a crystalline form of the present disclosure is not necessarily to have exactly the same X-ray diffraction pattern of the example shown herein. Any crystalline forms whose X-ray diffraction patterns have the same or similar characteristic peaks should be within the scope of the present disclosure. Those skilled in the art can compare the patterns shown in the present disclosure with that of an unknown crystalline form in order to identify whether these two groups of patterns reflect the same or different crystalline forms.

In some embodiments, Form CSII, Form CSIII and Form CSIV of the present disclosure are pure and substantially free of any other crystalline forms. In the present disclosure, the term "substantially free" when used to describe a novel crystalline form, means that the content of other crystalline forms in the novel crystalline form is less than 20% (w/w), specifically less than 10% (w/w), more specifically less than 5% (w/w) and furthermore specifically less than 1% (w/w).

In the present disclosure, the term "about" when referring to a measurable value such as weight, time, temperature, and the like, is meant to encompass variations of ± 10%, ± 5%, ± 1%, ± 0.5%, or even ± 0.1% of the specified amount.

Unless otherwise specified, the following examples were conducted at room temperature. According to the present disclosure, Compound I and/or its salt used as a raw material is solid (crystalline or amorphous), oil, liquid form or solution. Preferably, Compound I used as a raw material is a solid.

Raw materials of Compound I and/or a salt thereof used in the following examples were prepared by known methods in the prior art, for example, the method disclosed in WO2016196840A1.

### Example 1: Preparation of Form CSII

1000.0 mg of Compound I solid was weighed into a 20mL glass vial, followed by the addition of 10.0 mL of ethanol. After magnetic stirring at 50 °C, 500rpm for about 7 days, the obtained suspension was stood for 10 days at room temperature. Then a solid was isolated by suction filtration. After vacuum drying at 50 °C for about 3 hours, vacuum drying at 60 °C for about 17 hours, vacuum drying at 75 °C for about 5 hours, the obtained solid was confirmed, as determined by XPRD, to be Form CSII of the present disclosure, for which the XRPD pattern is as depicted in Figure 1, and the XRPD data are listed in Table 2.

The TGA curve is as depicted in Figure 2, which shows about 0.1% weight loss when heated from 26 °C to 100 °C.

The DSC curve is as depicted in Figure 3. It shows one endothermic peak at around 131 °C (onset temperature), which is the melting endothermic peak of Form CSII.

**Table 2**

| Diffraction angle 2θ (°) | d spacing (Å) | Relative intensity (%) |
|---|---|---|
| 4.12 | 21.45 | 19.88 |
| 8.22 | 10.75 | 8.22 |
| 10.20 | 8.67 | 76.59 |
| 10.75 | 8.23 | 21.41 |
| 11.25 | 7.86 | 58.69 |
| 12.32 | 7.18 | 3.73 |
| 12.70 | 6.97 | 4.02 |
| 13.53 | 6.54 | 7.76 |
| 14.57 | 6.08 | 2.63 |
| 15.50 | 5.72 | 10.22 |
| 15.72 | 5.64 | 17.09 |
| 16.54 | 5.36 | 26.47 |
| 17.77 | 4.99 | 29.30 |
| 18.97 | 4.68 | 2.69 |
| 20.48 | 4.34 | 52.71 |
| 20.66 | 4.30 | 46.45 |
| 21.19 | 4.19 | 17.95 |
| 22.62 | 3.93 | 100.00 |
| 23.35 | 3.81 | 12.36 |
| 23.61 | 3.77 | 12.14 |
| 24.65 | 3.61 | 21.65 |
| 25.13 | 3.54 | 16.59 |
| 25.64 | 3.47 | 12.38 |
| 26.28 | 3.39 | 3.58 |
| 26.72 | 3.34 | 5.17 |
| 27.28 | 3.27 | 6.45 |
| 29.49 | 3.03 | 1.47 |
| 30.97 | 2.89 | 4.55 |
| 31.51 | 2.84 | 3.56 |
| 32.67 | 2.74 | 2.50 |
| 33.65 | 2.66 | 1.56 |
| 34.51 | 2.60 | 1.94 |
| 35.29 | 2.54 | 2.67 |
| 36.57 | 2.46 | 1.95 |

### Example 2: NMR characterization of Form CSII

The ¹H NMR data of CSII are as follows: ¹H NMR (400 MHz, DMSO) δ 7.76 (d, J = 5.6 Hz, 1H), 7.53 - 7.37 (m, 4H), 7.22 (t, J = 10.6, 4.2 Hz, 1H), 7.14 (t, 4H), 6.97 (d, J = 5.5 Hz, 1H), 6.91 - 6.71 (m, 1H), 6.14 (dd, J = 16.8 Hz, 1H), 5.69 (dd, 1H), 4.81 (s, 2H), 4.51 (t, J = 13.4 Hz, 1H), 4.15 (dd, J = 34.1, 12.7 Hz, 2H), 3.76 (t, J = 12.1 Hz, 0.5H), 3.16 (t, J = 12.8 Hz, 0.5H), 2.83 - 2.61 (m, 0.5H), 2.46 - 2.30 (m, 1H), 2.03 - 1.77 (m, 2H), 1.66 - 1.45 (m, 1H). (According to the structure of Compound I, the peak of one hydrogen on the piperidine ring appears at δ 3.33-3.76 ppm. 0.5H spitted from this hydrogen is covered by the signal of water since it is close to the peak of water.)

### Example 3: Physical and chemical stability of Form CSII

Form CSII of the present disclosure and the prior art amorphous form were weighed and stored under 25 °C/60%RH, 40 °C/75%RH and 60 °C/75%RH conditions, in opened packages, respectively. The purity and solid form were determined by UPLC and XRPD. The results are listed in Table 3, and the overlaid XRPD patterns of Form CSII before and after storage for stability evaluation are shown in Figure 4.

**Table 3**

| Initial solid form | Storage condition | Storage time | Solid form | Purity | Purity change | The number of the purities exceeding the qualification thresholds |
|---|---|---|---|---|---|---|
| Form CSII | Initial | N/A | Form CSII | 99.86% | N/A | 0 |
| | 25 °C/60%RH | 6 months | Form CSII | 99.89% | +0.03% | 0 |
| | 40 °C/75%RH | 6 months | Form CSII | 99.87% | +0.01% | 0 |
| | 60 °C/75%RH | 1 month | Form CSII | 99.82% | -0.04% | 0 |
| Amorphou s | Initial | N/A | Amorpho us | 99.80% | N/A | 1 |
| | 25 °C/60%RH | 6 months | Amorpho us | 99.57% | -0.23% | 1 |
| | 40 °C/75%RH | 6 months | Amorpho us | 96.34% | -3.46% | 4 |
| | 60 °C/75%RH | 1 month | Amorpho us | 93.48% | -6.32% | 4 |

Remark: The qualification thresholds refer to INTERNATIONAL CONFERENCE ON HARMONISATION OF TECHNICAL REQUIREMENTS FOR REGISTRATION OF PHARMACEUTICALS FOR HUMAN USE, IMPURITIES IN NEW DRUG SUBSTANCES Q3A (R2).

The dose of Compound I is 60 mg once daily.

The results show that Form CSII was stable for at least 6 months under 25 °C/60%RH and 40 °C/75%RH conditions, and the solid form and purity remained basically unchanged, indicating Form CSII has good stability under both long-term and accelerated conditions. After storage under 60 °C/75%RH condition for 1 month, the solid form and purity remained basically unchanged, indicating Form CSII has good stability under stressed condition as well. The impurity contents of Form CSII did not exceed the qualification thresholds throughout the stability investigation processes, which meets the requirements of pharmaceutical development. After storage at 25 °C/60%RH, 40 °C/75%RH and 60 °C/75%RH, the purity of the prior art amorphous form decreased significantly, which is far below the requirements of pharmaceutical development. Particularly, after storage at 40 °C/75%RH for 6 months, the purity decreased by 3.46%, and the number of the impurities exceeding the qualification thresholds increased to four. After storage at 60 °C/75%RH for only 1 month, the purity decreased over 6.3%, and the number of the impurities exceeding the qualification thresholds increased to four. The results indicate that Form CSII of the present disclosure has outstanding chemical stability when compared with the prior art amorphous form.

### Example 4: Stability of Form CSII at high temperature

Certain amounts of Form CSII of the present disclosure and the prior art amorphous form were weighted and stored at 80 °C for 2 days, and the purities of the solids before and after storage were determined by UPLC, the results are as shown in Table 4.

**Table 4**

| Initial solid form | Package condition | Purity change |
|---|---|---|
| Form CSII | Glass vial with cap | +0.01% |
| Amorphous | Glass vial with cap | -1.16% |

The results indicate that the chemical purity of Form CSII basically remained unchanged for 2 days at 80 °C, while significant degradation of the amorphous form was observed under the same condition. Form CSII of the present disclosure has superior stability at high temperature compared with the prior art amorphous form.

### Example 5: Light stability of Form CSII

Certain amounts of Form CSII of the present disclosure and the prior art amorphous form were weighted and stored for about 1 week, in accordance with the method of the Chinese Pharmacopoeia, under the condition that the total illumination of the light source was not less than 1.2 × 106 lux-hr and the energy of the near-ultraviolet lamp was not less than 200 W-hr/m², the purities of the solids before and after storage were measured by UPLC and the results are shown in Table 5.

**Table 5**

| Initial solid form | Storage time | Purity change |
|---|---|---|
| Form CSII | 1 week | -0.02% |
| Amorphous | 1 week | -0.11% |

The results indicate that the chemical purity of Form CSII basically remained unchanged under the above light condition, while significant degradation of the amorphous form was observed under the same condition. Form CSII of the present disclosure has superior light stability compared with the prior art amorphous form.

### Example 6: Hygroscopicity of Form CSII

Certain amounts of Form CSII of the present disclosure and the prior art amorphous form were sampled for hygroscopicity tests using DVS instrument. The weight change at each relative humidity was recorded during the cycle of 0%RH-95%RH-0%RH at 25 °C.

The results are listed in Table 6, and the overlaid XRPD patterns of Form CSII before and after DVS test are shown in Figure 5.

**Table 6**

| Form | Weight gain at 80%RH |
|---|---|
| Form CSII | 0.60% |
| The prior art amorphous form | 3.69% |

The results show that Form CSII was slightly hygroscopic with a weight gain of 0.60% at 80%RH, while the prior art solid was hygroscopic with a weight gain of 3.69% at 80%RH. The hygroscopicity of Form CSII is superior to that of the prior art solid. In addition, the crystalline state of Form CSII remained unchanged after the DVS test, indicating that Form CSII has good stability.

Description and definition of hygroscopicity (general notice 9103 drug hygroscopicity test guidelines in 2020 edition of Chinese Pharmacopoeia, experimental condition: 25±1 °C, 80±2%RH):
Deliquescent: sufficient water is absorbed to form a solution.
Very hygroscopic: increase in mass is equal to or greater than 15.0 percent.
Hygroscopic: increase in mass is less than 15.0 percent but equal to or greater than 2.0 percent.
Slightly hygroscopic: increase in mass is less than 2.0 percent but equal to or greater than 0.2 percent.
Non hygroscopic or almost non hygroscopic: increase in mass is less than 0.2 percent.

(The definition of hygroscopicity in 5.11 of the 10^{th} version of European Pharmacopoeia is similar to the Chinese Pharmacopoeia.)

### Example 7: Grinding stability of Form CSII

Form CSII was grounded manually for 5 minutes in a mortar. XRPD was tested before and after the grinding. The results show that the crystalline state Form CSII remained unchanged after grinding, indicating that Form CSII has good grinding stability.

### Example 8: Preparation of the formulation comprising Form CSII

According to the formulation and process in Table 7 and Table 8, the tablets were prepared with an appropriate amount of Form CSII of the present disclosure. XRPD was tested before and after formulation. The results show that the crystalline state Form CSII remained unchanged after formulation process.

**Table 7**

| No. | Component | mg/unit | % (w/w) |
|---|---|---|---|
| 1 | Compound I | 20 | 20 |
| 2 | Microcrystalline Cellulose | 69.5 | 69.5 |
| 3 | Hydroxypropyl methyl cellulose | 3.0 | 3.0 |
| 4 | Crospovidone | 6.0 | 6.0 |
| 5 | Colloidal silicon dioxide | 0.5 | 0.5 |
| 6 | Magnesium stearate | 1.0 | 1.0 |
| Total | | 100.0 | 100.0 |

**Table 8**

| Stage | Procedure |
|---|---|
| Blending | According to the formulation, materials of Nos. 1-6 were weighed into a glass vial and blended manually for 2 minutes. |
| Tableting | The mixed powders were tableted by the ENERPAC single punch manual tablet press equipped with a die of ϕ 9*4 mm (tablet weight: 100 ± 10 mg; pressure: 5 ±1 KN). |

### Example 9: Preparation of Form CSIII

491.9 mg of Compound I solid was weighed into a 20mL glass vial, followed by the addition of 5 mL of acetone. The obtained suspension was stirred at room temperature for about 15 minutes, then 3 mL of acetone was added. After stirring for about 4 days at 5°C, a solid was isolated. After vacuum drying at 50 °C for about 20 hours, the obtained solid was confirmed, as determined by XRPD, to be Form CSIII of the present disclosure, for which the XRPD pattern is as depicted in Figure 6, and the XRPD data are listed in Table 9.

The TGA curve is as depicted in Figure 7, which shows about 0.5% weight loss when heated from 26 °C to 100 °C.

The DSC curve is as depicted in Figure 8. It shows one endothermic peak at around 133 °C (onset temperature), which is the melting endothermic peak of Form CSIII.

**Table 2**

| Diffraction angle 2θ (°) | d spacing (Å) | Relative intensity (%) |
|---|---|---|
| 4.21 | 20.97 | 17.43 |
| 8.41 | 10.51 | 12.60 |
| 10.43 | 8.48 | 30.13 |
| 11.05 | 8.00 | 100.00 |
| 12.62 | 7.01 | 2.96 |
| 13.26 | 6.68 | 15.60 |
| 14.40 | 6.15 | 3.94 |
| 16.27 | 5.45 | 26.01 |
| 16.57 | 5.35 | 11.24 |
| 17.74 | 5.00 | 31.55 |
| 19.08 | 4.65 | 9.23 |
| 19.51 | 4.55 | 6.10 |
| 20.62 | 4.31 | 48.54 |
| 20.95 | 4.24 | 50.67 |
| 21.69 | 4.10 | 22.41 |
| 22.22 | 4.00 | 43.04 |
| 23.10 | 3.85 | 27.39 |
| 23.82 | 3.74 | 14.49 |
| 24.19 | 3.68 | 20.11 |
| 25.05 | 3.56 | 17.27 |
| 25.41 | 3.50 | 28.97 |
| 25.97 | 3.43 | 6.03 |
| 26.50 | 3.36 | 2.71 |
| 27.25 | 3.27 | 6.41 |
| 28.14 | 3.17 | 3.41 |
| 29.63 | 3.01 | 2.67 |
| 31.27 | 2.86 | 6.81 |
| 32.82 | 2.73 | 5.09 |
| 34.85 | 2.57 | 4.13 |
| 35.98 | 2.50 | 2.34 |
| 37.48 | 2.40 | 2.38 |

### Example 10: NMR characterization of Form CSIII

The ¹H NMR data of CSIII are as follows: ¹H NMR (400 MHz, DMSO) δ 7.76 (d, J = 5.6 Hz, 1H), 7.52 - 7.36 (m, 4H), 7.21 (t, 1H), 7.14 (t, 4H), 6.97 (d, J = 5.5 Hz, 1H), 6.90 - 6.72 (m, 1H), 6.14 (dd, J = 17.0 Hz, 1H), 5.69 (dd, J = 13.6 Hz, 1H), 4.81 (s, 2H), 4.51 (t, J = 13.5 Hz, 1H), 4.14 (dd, J = 33.1, 14.1 Hz, 2H), 3.76 (t, J = 12.1 Hz, 0.5H), 3.16 (t, J = 12.6 Hz, 0.5H), 2.82 - 2.59 (m, 0.5H), 2.44 - 2.28 (m, 1H), 2.11 - 1.75 (m, 2H), 1.68 - 1.37 (m, 1H). (According to the structure of Compound I, the peak of one hydrogen on the piperidine ring appears at δ 3.33-3.76 ppm. 0.5H spitted from this hydrogen is covered by the signal of water since it is close to the peak of water.)

### Example 11: Physical and chemical stability of Form CSIII

Form CSIII of the present disclosure and the prior art amorphous form were weighed and stored under 25 °C/60%RH, 40 °C/75%RH and 60 °C/75%RH conditions, in opened packages, respectively. The purity and solid form were determined by UPLC and XRPD. The results are listed in Table 10, and the overlaid XRPD patterns of Form CSIII before and after storage for stability evaluation are shown in Figure 9.

**Table 3**

| Initial solid form | Storage condition | Storage time | Solid form | Purity | Purity change | The number of the purities exceeding the qualification thresholds |
|---|---|---|---|---|---|---|
| Form CSIII | Initial | N/A | Form CSIII | 99.91% | N/A | 0 |
| | 25 °C/60%RH | 6 months | Form CSIII | 99.93% | +0.02% | 0 |
| | 40 °C/75%RH | 6 months | Form CSIII | 99.91% | 0% | 0 |
| | 60 °C/75%RH | 1 month | Form CSIII | 99.86% | -0.05% | 0 |
| Amorphous | Initial | N/A | Amorphous | 99.80% | N/A | 1 |
| | 25 °C/60%RH | 6 months | Amorphous | 99.57% | -0.23% | 1 |
| | 40 °C/75%RH | 6 months | Amorphous | 96.34% | -3.46% | 4 |
| | 60 °C/75%RH | 1 month | Amorphous | 93.48% | -6.32% | 4 |

Remark: The qualification thresholds refer to INTERNATIONAL CONFERENCE ON HARMONISATION OF TECHNICAL REQUIREMENTS FOR REGISTRATION OF PHARMACEUTICALS FOR HUMAN USE, IMPURITIES IN NEW DRUG SUBSTANCES Q3A (R2).

The dose of Compound I is 60 mg once daily.

The results show that Form CSIII was stable for at least 6 months under 25 °C/60%RH and 40 °C/75%RH conditions, and the solid form and purity remained basically unchanged, indicating Form CSIII has good stability under both long-term and accelerated conditions. After storage under 60 °C/75%RH condition for 1 month, the solid form and purity remained basically unchanged, indicating Form CSIII has good stability under stressed condition as well. The impurity contents of Form CSIII did not exceed the qualification thresholds throughout the stability investigation processes, which meets the requirements of pharmaceutical development. After storage at 25 °C/60%RH, 40 °C/75%RH and 60 °C/75%RH, the purity of the prior art amorphous form decreased significantly, which is far below the requirements of pharmaceutical development. Particularly, after storage at 40 °C/75%RH for 6 months, the purity decreased by 3.46%, and the number of the impurities exceeding the qualification thresholds increased to four. After storage at 60 °C/75%RH for only 1 month, the purity decreased over 6.3%, and the number of the impurities exceeding the qualification thresholds increased to four. The results indicate that Form CSIII of the present disclosure has outstanding chemical stability when compared with the prior art amorphous form.

### Example 12: Stability of Form CSIII at high temperature

Certain amounts of Form CSIII of the present disclosure and the prior art amorphous form were weighted and stored at 80 °C for 2 days, and the purities of the solids before and after storage were determined by UPLC, the results are as shown in Table 11.

**Table 11**

| Initial solid form | Package condition | Purity change |
|---|---|---|
| Form CSIII | Glass vial with cap | +0.04% |
| Amorphous | Glass vial with cap | -1.16% |

The results indicate that the chemical purity of Form CSIII basically remained unchanged for 2 days at 80 °C, while significant degradation of the amorphous form was observed under the same condition. Form CSIII of the present disclosure has superior stability at high temperature compared with the prior art amorphous form.

### Example 13: Light stability of Form CSIII

Certain amounts of Form CSIII of the present disclosure and the prior art amorphous form were weighted and stored for about 1 week, in accordance with the method of the Chinese Pharmacopoeia, under the condition that the total illumination of the light source was not less than 1.2 × 10⁶ lux-hr and the energy of the near-ultraviolet lamp was not less than 200 W-hr/m², the purities of the solids before and after storage were measured by UPLC and the results are shown in Table 12.

**Table 12**

| Initial solid form | Storage time | Purity change |
|---|---|---|
| Form CSIII | 1 week | +0.02% |
| Amorphous | 1 week | -0.11% |

The results indicate that the chemical purity of Form CSIII basically remained unchanged under the above light condition, while significant degradation of the amorphous form was observed under the same condition. Form CSIII of the present disclosure has superior light stability compared with the prior art amorphous form.

### Example 14: Hygroscopicity of Form CSIII

Certain amounts of Form CSIII of the present disclosure and the prior art amorphous form were sampled for hygroscopicity tests using DVS instrument. The weight change at each relative humidity was recorded during the cycle of 0%RH-95%RH-0%RH at 25 °C.

The results are listed in Table 13, and the overlaid XRPD patterns of Form CSIII before and after DVS test are shown in Figure 10.

**Table 13**

| Form | Weight gain at 80%RH |
|---|---|
| Form CSIII | 0.66% |
| The prior art amorphous form | 3.69% |

The results show that Form CSIII was slightly hygroscopic with a weight gain of 0.66% at 80%RH, while the prior art solid was hygroscopic with a weight gain of 3.69% at 80%RH. The hygroscopicity of Form CSIII is superior to that of the prior art. In addition, the crystalline state of Form CSIII remained unchanged after the DVS test, indicating that Form CSIII has good stability.

### Example 15: Grinding stability of Form CSIII drug product

Form CSIII was grounded manually for 5 minutes in a mortar. XRPD was tested before and after the grinding. The results show that the crystalline state Form CSIII remained unchanged after grinding, indicating that Form CSIII has good grinding stability.

### Example 16: Preparation of the formulation comprising Form CSIII drug product

According to the formulation and process in Table 7 and Table 8, the tablets were prepared with an appropriate amount of Form CSIII of the present disclosure. XRPD was tested before and after formulation. The results show that the crystalline state Form CSIII remained unchanged after formulation process.

### Example 17: Preparation of Form CSIV

11.0 mg of Compound I solid was weighed into a glass vial, followed by the addition of 0.08 mL of metyl tert-butyl ether to form a suspension. The suspension was stirred at -20 °C for about 23 hours, then part of the solid was isolated. The obtained solid was confirmed, as determined by XRPD, to be Form CSIV of the present disclosure. Then additional 0.08mL of metyl tert-butyl ether was added into the vial. After stirring for about 2 days at room temperature, a solid was isolated. The obtained solid was confirmed, as determined by XRPD, to be Form CSIV of the present disclosure, for which the XRPD pattern is as depicted in Figure 11, and the XRPD data are listed in Table 14.

**Table 14**

| Diffraction angle 2θ (°) | d spacing (Å) | Relative intensity (%) |
|---|---|---|
| 7.67 | 11.52 | 9.38 |
| 8.46 | 10.45 | 5.92 |
| 12.67 | 6.99 | 18.31 |
| 12.91 | 6.86 | 53.50 |
| 13.23 | 6.69 | 28.96 |
| 13.55 | 6.54 | 17.05 |
| 13.83 | 6.40 | 20.08 |
| 14.84 | 5.97 | 6.62 |
| 15.44 | 5.74 | 17.23 |
| 15.65 | 5.66 | 25.58 |
| 17.20 | 5.15 | 36.78 |
| 17.41 | 5.09 | 19.37 |
| 18.16 | 4.89 | 10.35 |
| 18.62 | 4.77 | 40.46 |
| 19.10 | 4.65 | 30.25 |
| 19.75 | 4.49 | 9.11 |
| 20.22 | 4.39 | 13.92 |
| 20.78 | 4.27 | 15.87 |
| 21.13 | 4.21 | 22.64 |
| 21.97 | 4.05 | 100.00 |
| 22.26 | 3.99 | 17.61 |
| 22.65 | 3.93 | 12.38 |
| 23.25 | 3.83 | 48.16 |
| 23.62 | 3.77 | 17.01 |
| 24.27 | 3.67 | 6.04 |
| 24.86 | 3.58 | 18.52 |
| 25.21 | 3.53 | 9.81 |
| 25.50 | 3.49 | 18.08 |
| 25.98 | 3.43 | 13.68 |
| 26.43 | 3.37 | 15.53 |
| 26.68 | 3.34 | 26.72 |
| 26.97 | 3.31 | 14.65 |
| 28.49 | 3.13 | 1.86 |
| 29.95 | 2.98 | 14.94 |
| 30.87 | 2.90 | 2.85 |
| 31.70 | 2.82 | 3.93 |
| 32.44 | 2.76 | 3.97 |
| 32.93 | 2.72 | 6.38 |
| 33.63 | 2.67 | 1.65 |
| 35.22 | 2.55 | 2.37 |
| 35.83 | 2.51 | 2.54 |
| 37.64 | 2.39 | 2.32 |
| 38.68 | 2.33 | 1.29 |

### Example 18: Preparation of Form CSIV

300.0 mg of Compound I solid was weighed into a glass vial, followed by the addition of 4.5 mL of isopropylbenzene. After stirring at -20 °C for about 39 hours, a solid was isolated by filtration. After vacuum drying for 22 hours at 50°C, the obtained solid was confirmed, as determined by XRPD, to be Form CSIV of the present disclosure, for which the XRPD pattern is as depicted in Figure 12, and the XRPD data are listed in Table 15.

The DSC curve is as depicted in Figure 13. It shows one endothermic peak at around 144 °C (onset temperature), which is the melting endothermic peak of Form CSIV.

The ¹H NMR data of CSIV are as follows: ¹H NMR (400 MHz, DMSO) δ 7.76 (d, J = 5.6 Hz, 1H), 7.53 - 7.39 (m, 4H), 7.22 (t, J = 7.4 Hz, 1H), 7.14 (t, J = 7.7 Hz, 4H), 6.97 (d, J = 5.5 Hz, 1H), 6.92 - 6.74 (m, 1H), 6.14 (dd, 1H), 5.69 (dd, 1H), 4.82 (s, 2H), 4.52 (t, J = 11.8 Hz, 1H), 4.15 (dd, J = 33.2, 12.0 Hz, 2H), 3.78 (t, J = 12.8 Hz, 0.5H), 3.16 (t, J = 12.6 Hz, 0.5H), 2.79 - 2.63 (m, 0.5H), 2.40 - 2.25 (m, 1H), 2.07 - 1.76 (m, 2H), 1.68 - 1.40 (m, 1H). (According to the structure of Compound I, the peak of one hydrogen on the piperidine ring appears at δ 3.33-3.76 ppm. 0.5H spitted from this hydrogen is covered by the signal of water since it is close to the peak of water.)

**Table 15**

| Diffraction angle 2θ (°) | d spacing (Å) | Relative intensity (%) |
|---|---|---|
| 7.69 | 11.49 | 16.30 |
| 8.49 | 10.42 | 5.18 |
| 10.80 | 8.20 | 1.53 |
| 12.66 | 6.99 | 13.03 |
| 12.91 | 6.86 | 46.36 |
| 13.22 | 6.70 | 19.00 |
| 13.53 | 6.54 | 11.14 |
| 13.83 | 6.40 | 24.91 |
| 14.83 | 5.97 | 6.07 |
| 15.47 | 5.73 | 26.77 |
| 17.20 | 5.15 | 39.59 |
| 17.45 | 5.08 | 16.07 |
| 18.17 | 4.88 | 5.56 |
| 18.62 | 4.77 | 38.57 |
| 19.11 | 4.64 | 22.97 |
| 19.75 | 4.49 | 6.02 |
| 20.22 | 4.39 | 13.04 |
| 20.78 | 4.27 | 12.61 |
| 21.11 | 4.21 | 12.41 |
| 21.97 | 4.05 | 100.00 |
| 22.65 | 3.93 | 6.91 |
| 23.25 | 3.83 | 46.21 |
| 23.63 | 3.77 | 10.58 |
| 24.27 | 3.67 | 5.65 |
| 24.86 | 3.58 | 9.79 |
| 25.22 | 3.53 | 6.70 |
| 25.51 | 3.49 | 15.72 |
| 25.99 | 3.43 | 9.51 |
| 26.68 | 3.34 | 20.09 |
| 26.96 | 3.31 | 12.39 |
| 27.95 | 3.19 | 2.33 |
| 28.48 | 3.13 | 1.64 |
| 29.96 | 2.98 | 9.46 |
| 31.69 | 2.82 | 1.53 |
| 32.19 | 2.78 | 2.16 |
| 32.91 | 2.72 | 4.09 |
| 35.22 | 2.55 | 1.28 |
| 35.80 | 2.51 | 1.45 |
| 37.59 | 2.39 | 2.22 |
| 38.65 | 2.33 | 0.79 |

### Example 19: TGA test of Form CSIV

A certain amount of Form CSIV was sampled for TGA test. Result of TGA is shown in Figure 14, showing about 0.2% weight loss when heated from 29 °C to 120 °C.

### Example 20: Physical and chemical stability of Form CSIV

Form CSIV of the present disclosure and the prior art amorphous form were weighed and stored under 25 °C/60%RH and 40 °C/75%RH conditions, in opened packages, respectively. The purity and solid form were determined by UPLC and XRPD. The results are listed in Table 16, and the overlaid XRPD patterns of Form CSIV before and after storage for stability evaluation are shown in Figure 15.

**Table 4**

| Initial solid form | Storage condition | Storage time | Solid form | Purity | Purity change |
|---|---|---|---|---|---|
| Form CSIV | Initial | N/A | Form CSIV | 99.74% | N/A |
| | 25 °C/60%RH | 2 months | Form CSIV | 99.74% | 0.00% |
| | 40 °C/75%RH | 2 months | Form CSIV | 99.75% | +0.01% |
| Amorphous | Initial | N/A | Amorphous | 99.80% | N/A |
| | 40 °C/75%RH | 2 months | Amorphous | 97.62% | -2.18% |

Remark: The qualification thresholds refer to INTERNATIONAL CONFERENCE ON HARMONISATION OF TECHNICAL REQUIREMENTS FOR REGISTRATION OF PHARMACEUTICALS FOR HUMAN USE, IMPURITIES IN NEW DRUG SUBSTANCES Q3A (R2).

The dose of Compound I is 60 mg once daily.

The results show that Form CSIV was stable for at least 2 months under 25 °C/60%RH and 40 °C/75%RH conditions, and the solid form and purity remained basically unchanged, indicating Form CSIV has good stability under both long-term and accelerated conditions. After storage at 40 °C/75%RH, the purity of the prior art solid decreased significantly, the purity decreased by 2.18%. The results indicate that Form CSIV of the present disclosure has outstanding chemical stability when compared with the prior art amorphous form.

### Example 21: Stability of Form CSIV at high temperature

Certain amounts of Form CSIV of the present disclosure and the prior art amorphous form were stored at 80 °C for 2 days, and the purities of the solids before and after storage were determined by UPLC, the results are as shown in Table 17.

**Table 17**

| Initial solid form | Package condition | Purity change |
|---|---|---|
| Form CSIV | Glass vial with cap | +0.03% |
| Amorphous | Glass vial with cap | -1.16% |

The results indicate that the chemical purity of Form CSIV basically remained unchanged for 2 days at 80 °C, while significant degradation of the amorphous form was observed under the same condition. Form CSIV of the present disclosure has superior stability at high temperature compared with the prior art amorphous form.

### Example 22: Light stability of Form CSIV

Certain amounts of Form CSIV of the present disclosure and the prior art amorphous form were weighted and stored for about 1 week, in accordance with the method of the Chinese Pharmacopoeia, under the condition that the total illumination of the light source was not less than 1.2 × 10⁶ lux-hr and the energy of the near-ultraviolet lamp was not less than 200 W-hr/m², the purities of the solids before and after storage were measured by UPLC and the results are shown in Table 18.

**Table 18**

| Initial solid form | Storage time | Purity change |
|---|---|---|
| Form CSIV | 1 week | +0.06% |
| Amorphous | 1 week | -0.11% |

The results indicate that the chemical purity of Form CSIV basically remained unchanged under the above light condition, while significant degradation of the amorphous form was observed under the same condition. Form CSIV of the present disclosure has superior light stability compared with the prior art amorphous form.

### Example 23: Hygroscopicity of Form CSIV

Certain amounts of Form CSIV of the present disclosure and the prior art amorphous form were sampled for hygroscopicity tests using DVS instrument. The weight change at each relative humidity was recorded during the cycle of 0%RH-95%RH-0%RH at 25 °C.

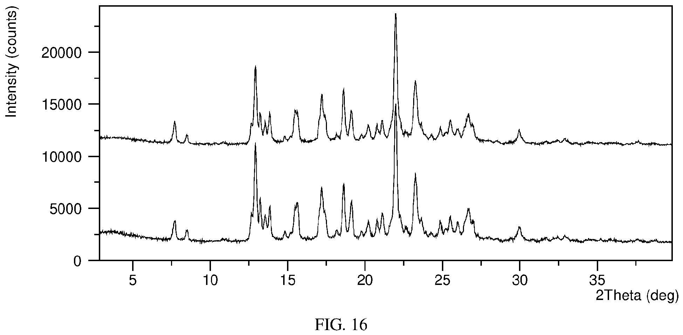

The results are listed in Table 19, and the overlaid XRPD patterns of Form CSIV before and after DVS test are shown in Figure 16.

**Table 19**

| Form | Weight gain at 80%RH |
|---|---|
| Form CSIV | 0.24% |
| Prior art solid | 3.69% |

The results show that Form CSIV was slightly hygroscopic with a weight gain of 0.24% at 80%RH, while prior art amorphous form was hygroscopic with a weight gain of 3.69% at 80%RH. The hygroscopicity of Form CSIV is superior to that of the prior art. In addition, the crystalline state of Form CSIV remained unchanged after the DVS test, indicating that Form CSIV has good stability.

### Example 24: Grinding stability of Form CSIV

Form CSIV was grounded manually for 5 minutes in a mortar. XRPD was tested before and after the grinding. The results show that the crystalline state Form CSIV remained unchanged after grinding, indicating that Form CSIV has good grinding stability.

### Example 25: Preparation of the formulation comprising Form CSIV

According to the formulation and process in Table 7 and Table 8, the tablets were prepared with an appropriate amount of Form CSIV of the present disclosure. XRPD was tested before and after formulation. The results show that the crystalline state Form CSIV remained unchanged after formulation process.

The examples described above are only for illustrating the technical concepts and features of the present disclosure and intended to make those skilled in the art being able to understand the present disclosure and thereby implement it and should not be concluded to limit the protective scope of this disclosure. Any equivalent variations or modifications according to the spirit of the present disclosure should be covered by the protective scope of the present disclosure.

## Claims

1. A crystalline form of Compound I, wherein the X-ray powder diffraction pattern comprises characteristic peaks at 2theta values of 4.1°±0.2°, 10.2°±0.2° and 22.6°±0.2° using CuKα radiation.

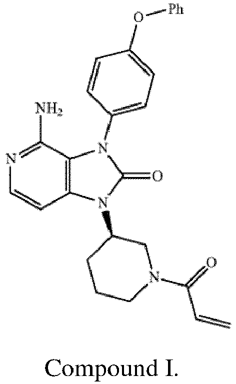

2. The crystalline form of Compound I according to claim 1, wherein the X-ray powder diffraction pattern comprises at least one characteristic peak at 2theta values of 11.3°±0.2°, 16.5°±0.2°and 17.8°±0.2° using CuKα radiation.

3. The crystalline form of Compound I according to claim 1, wherein the X-ray powder diffraction pattern comprises at least one characteristic peak at 2theta values of 8.2°±0.2°, 10.8°±0.2°and 24.7°±0.2°using CuKα radiation.

4. The crystalline form of Compound I according to claim 2, wherein the X-ray powder diffraction pattern comprises at least one characteristic peak at 2theta values of 8.2°±0.2°, 10.8°±0.2°and 24.7°±0.2°using CuKα radiation.

5. The crystalline form of Compound I according to claim 1, wherein the X-ray powder diffraction pattern is substantially as depicted in Figure 1 using CuKα radiation.

6. A process for preparing crystalline form according to claim 1, wherein the process comprises: adding the solid of Compound I into an alchol solvent to form a suspension, stirring, and separating to obtain a solid, high temperature vaccum drying the solid to obtain the crystalline form.

7. The process according to Claim 6, wherein said alcohol solvent is an alcohol of C1-C4, and a temperature of stirring is 0-50°C.

8. A crystalline form of Compound I, wherein the X-ray powder diffraction pattern comprises characteristic peaks at 2theta values of 4.2°±0.2°, 11.1°±0.2° and 21.7°±0.2° using CuKα radiation.

9. The crystalline form of Compound I according to claim 8, wherein the X-ray powder diffraction pattern comprises at least one characteristic peak at 2theta values of 20.6°±0.2°, 21.0°±0.2° and 22.2°±0.2° using CuKα radiation.

10. The crystalline form of Compound I according to claim 8, wherein the X-ray powder diffraction pattern comprises at least one characteristic peak at 2theta values of 10.4°±0.2°, 17.7°±0.2° and 23.1°±0.2°using CuKα radiation.

11. The crystalline form of Compound I according to claim 9, wherein the X-ray powder diffraction pattern comprises at least one characteristic peak at 2theta values of 10.4°±0.2°, 17.7°±0.2° and 23.1°±0.2°using CuKα radiation.

12. The crystalline form of Compound I according to claim 8, wherein the X-ray powder diffraction pattern is substantially as depicted in Figure 6 using CuKα radiation.

13. A process for preparing crystalline form according to claim 8, wherein the process comprises: adding the solid of Compound I into acetone to form a suspension, then stirring to obtain the crystalline form.

14. The process according to Claim 13, wherein a temperature of stirring is 0-50°C.

15. A crystalline form of Compound I, wherein the X-ray powder diffraction pattern comprises characteristic peaks at 2theta values of 8.5°±0.2°, 18.6°±0.2° and 22.0°±0.2° using CuKα radiation.

16. The crystalline form of Compound I according to claim 15, wherein the X-ray powder diffraction pattern comprises at least one characteristic peak at 2theta values of 12.9°±0.2°, 19.1°±0.2° and 23.3°±0.2° using CuKα radiation.

17. The crystalline form of Compound I according to claim 15, wherein the X-ray powder diffraction pattern comprises at least one characteristic peak at 2theta values of 13.2°±0.2°, 13.8°±0.2°and 21.1°±0.2° using CuKα radiation.

18. The crystalline form of Compound I according to claim 16, wherein the X-ray powder diffraction pattern comprises at least one characteristic peak at 2theta values of 13.2°±0.2°, 13.8°±0.2° and 21.1°±0.2° using CuKα radiation.

19. The crystalline form of Compound I according to claim 15, wherein the X-ray powder diffraction pattern is substantially as depicted in Figure 11 using CuKα radiation.

20. A process for preparing crystalline form according to claim 15, wherein the process comprises: adding the solid of Compound I into an ether solvent or an aromatic hydrocarbon solvent to form a suspension, stirring at -20°C -5°C to obtain the crystalline form.

21. The process according to Claim 20, wherein said ether solvent is an ether of C5, and aromatic hydrocarbon solvent is an aromatic hydrocarbon solvent of C9, the temperature of said stirring is -20°C.

22. A pharmaceutical composition, wherein said pharmaceutical composition comprises a therapeutically effective amount of the crystalline form according to claim 1, or the crystalline form according to claim 8, or the crystalline form according to claim 15, or any mixture of any two of the crystalline forms, or any mixture of the three crystalline forms; and pharmaceutically acceptable excipients.

23. Use of the crystalline form according to claim 1, or the crystalline form according to claim 8, or the crystalline form according to claim 15, or any mixture of any two of the crystalline forms, or any mixture of the three crystalline forms for the preparation of a BTK inhibitor drug.

24. Use of the crystalline form according to claim 1, or the crystalline form according to claim 8, or the crystalline form according to claim 15, or any mixture of any two of the crystalline forms, or any mixture of the three crystalline forms for the preparation of a drug for the treatment of multiple sclerosis.
